# EUROPÄISCHE PATENTSCHRIFT

(11) **EP 0 710 113 B1**
(45) Veröffentlichungstag und Bekanntmachung des Hinweises auf die Patenterteilung: **10.06.1998**
(21) Anmeldenummer: 94922222.8
(22) Anmeldetag: 19.07.1994
(51) Int. Cl.: A61K 33/26, A61K 7/48, A61K 7/42

(54) **PRÄPARAT ZUR DURCHBLUTUNGSFÖRDERUNG DAS HARTMAGNETISCHE TEILCHEN ENTHÄLT**
PREPARATION FOR IMPROVING THE BLOOD SUPPLY CONTAINING HARD MAGNETIC PARTICLES
PREPARATION FAVORISANT L'IRRIGATION SANGUINE CONTENANT DES PARTICULES FORTEMENT MAGNETIQUES

(30) Priorität: 19.07.1993 DE 4325071
(43) Veröffentlichungstag der Anmeldung: 08.05.1996
(73) Patentinhaber: LANCASTER GROUP GmbH, 67059 Ludwigshafen (DE)
(72) Erfinder: ZASTROW, Leonhard, MC-98000 Monaco (MC); HÜLSENBERG, Dagmar, D-98693 Ilmenau (DE); GOLZ, Karin, D-13137 Berlin (DE); STANZL, Klaus, White Plains, NY 10605 (US)
(74) Vertreter: Walter, Wolf-Jürgen
(86) Internationale Anmeldenummer: DE9400879
(87) Internationale Veröffentlichungsnummer: WO9503061

(56) Entgegenhaltungen:
- PATENT ABSTRACTS OF JAPAN vol. 16, no. 356 (C-969) (5399) 31. Juli 1992 & JP,A,04 108 710 (YOKO SHIGA) 9. April 1992 in der Anmeldung erwähnt

## Beschreibung

Die Erfindung betrifft ein Präparat zur Durchblutungsförderung.

Die Förderung der Durchblutung der Haut ist eine Aufgabe, für deren Lösung bereits eine Reihe von Forschungsergebnissen bekanntgeworden und bei der unterschiedliche Wege eingeschlagen worden sind. Dabei ist insbesondere in den letzten Jahren auch der Einfluß magnetischer Kräfte verstärkt untersucht worden. Es wurde u.a. der Einsatz gepulster elektromagnetischer Felder als potentiell nützliche Therapie für die postchirurgische Schmerz- und Ödembehandlung in einer Studie untersucht (Mayrovitz, H.N., Larsen, P.B., WOUNDS, Vol.4, Nr.5, 197 (1992).

Aus Beauty Forum 2/93, Seite 46 ist der Einsatz eines Stiftes bekannt, den der Anwender über die Haut gleiten läßt, und bei dem ein Magnetfeld auf die Hautoberfläche einwirkt. Nach Aussage des Herstellers sollen nicht mehr voll funktionsfähige Zellen auf Basis eines Magnetfeldes stimuliert werden, wodurch ein Selbstheilungsprozeß in Gang gesetzt und die Haut wieder fest und elastisch werden soll.

Weiterhin wurde der Einsatz magnetischer polymerer Teilchen, an die teilweise pharmakologisch wirksame Verbindungen angekoppelt sind, z.B. in der US-A-4501726, US-A-4335094 und US-A-5039559 beschrieben. In diesen Patentbeschreibungen wurden weichmagnetische Ferrit-Teilchen oder Ferro-Aluminate mit polymeren Materialien verkapselt und in den Körper eingebracht.

Ein magnetisches kosmetisches Präparat ist in der JP-A-4-108710 (Yoko Shiga) beschrieben. Dort werden ferromagnetische Substanzen wie z.B. Magnetit, Mangan-Zink-Ferrit (alles weichmagnetische Ferrite) in entmagnetisiertem Zustand in einem kosmetischen Präparat verteilt und das Präparat nach der kosmetischen Anwendung auf der Haut magnetisiert. Diese Anwendungsform soll eine durchblutungsfördernde Wirkung haben, im Tierversuch bei 0,1 % Magnetitanteil eine um 3,4 % erhöhte Durchblutung. Weitere Angaben sind in der Schrift nicht enthalten.

Der Erfindung liegt die Aufgabe zugrunde, ein neues Präparat mit wesentlich verbesserter durchblutungsfördernder Wirkung bereitzustellen.

Erfindungsgemäß enthält das Präparat zur Durchblutungsförderung einen pharmazeutisch oder kosmetisch annehmbaren Trägerstoff sowie gegebenenfalls für die Formulierung übliche Additive und darin fein verteilte hartmagnetische Einbereichsteilchen mit hoher Koerzitivfeldstärke und mit Korngrößen im Bereich von 600 bis 1200 nm.

Unter dem Begriff "Einbereichsteilchen" werden Einkristalle mit von Hause aus einheitlicher magnetischer Orientierung verstanden. Besonders bevorzugt in der vorliegenden Erfindung als hartmagnetische Einbereichsteilchen sind Barium- oder Strontiumhexaferrite, die vorteilhafterweise nicht dotiert sind. Die Herstellung dieser undotierten Barium- oder Strontiumhexaferrite erfolgt nach bekannten Verfahren, z.B. nach der Glaskristallisationstechnik durch Züchtung von Einkristallen aus einer abgeschreckten Glasschmelze. Ein geeignetes Glas dafür befindet sich im Dreistoffsystem BaO-Fe₂O₃-B₂O₃. Es setzt sich vorteilhaft zusammen aus 20 bis 50 Gew.-% Fe₂O₃, 30 bis 50 Gew.-% BaO und 20 bis 50 Gew.-% B₂O₃.

Das Durchmesser/Dickenverhältnis der Kristalle von Bariumhexaferrit oder Strontiumhexaferrit liegt im allgemeinen bei 3 : 1 bis 10 : 1.

Die Korngrößen der Einbereichsteilchen liegen vorzugsweise im Bereich von 750 bis 1000 nm, insbesondere im Bereich von 800 bis 950 nm. In diesem Bereich haben die Teilchen ein besonders vorteilhafte große Koerzitivfeldstärke. Die Koerzitivfeldstärke liegt vorteilhaft im Bereich von 3000 bis 5000 Oersted, vorzugsweise im Bereich von 4000 bis 5000 Oersted; sie kann jedoch auch darüber liegen.

Die erfindungsgemäßen Einbereichsteilchen lassen sich in einem pharmazeutisch/kosmetisch annehmbaren Trägerstoff und in Additiven mit den üblichen verfahren sehr gut dispergieren, und es kommt in der Dispersion zu keinen oder nur unwesentlichen Aggregationen. Dies ist besonders überraschend, da aus allen Veröffentlichungen des Standes der Technik hervorgeht, daß dauermagnetische d.h. hartmagnetische Teilchen stets zur Aggregation neigen und daher mit bestimmten organischen Polymeren oder anorganischen Stoffen in eine Dispersion eingebracht werden müssen, wobei die Funktion dieser anorganischen oder organischen Zusätze als eine Gittersubstanz, in die die hartmagnetischen Teilchen eingelagert werden, oder infolge Ankopplung an diese Zusatzstoffe erfolgt und damit eine Aggregation vermieden wird. Dies ist in der vorliegenden Erfindung nicht erforderlich, und man erhält allein mit den hartmagnetischen Einbereichsteilchen, insbesondere mit den nach der Glaskristallisationstechnik hergestellten Barium- oder Strontiumhexaferriten eine Dispersion, die gegebenenfalls unter Zusatz bestimmter Dispergierhilfsmittel problemlos eine stabile Dispersion ergibt.

Die neuen Präparate zeigen eine ausgezeichnete durchblutungsfördernde Wirkung. Sie könner z.B. mit Hilfe üblicher kosmetischer oder dermatologischer Trägerstoffe zu einem auf die Haut aufzutragenden Mittel verarbeitet werden oder auch in enterale oder parenterale Verarbeitungsformen nach üblichen Techniken und mit den üblichen Trägersystemen überführt werden. Beim Einsatz auf der Haut werden beispielsweise die beim medizinischen Gebrauch herkömmlicher Vasodilatantien auftretenden Rötungen oder Hautirritationen und Augenreizungen vermieden, was einen erheblichen Vorteil in der praktischen Anwendung dermatologischer Präparate darstellt. Infolge der durchblutungsfördernden Wirkung ist auch eine Haarwuchs-stimuliernde Wirksamkeit bei entsprechenden Konzentrationen zu beobachten.

Der Anteil der erfindungsgemäßen hartmagnetischen Einbereichsteilchen in der Dispersion liegt im allgemeinen im Bereich von 0,01 bis 70 Gew.-%, vorzugsweise im Bereich von 0,01 bis 15 Gew.-%, insbesondere im Bereich von 0,01 bis 10 Gew.-%, bezogen auf das Gesamtgewicht der Dispersion.

Erfindungsgemäß besonders vorteilhaft für die dermale/kosmetische Anwendung ist es, wenn die hartmagnetischen Einbereichsteilchen in Kombination mit asymmetrischen lamellaren Aggregaten vorliegen, die aus Phospholipiden mit einem Phosphatidylcholingehalt im Bereich von 30 bis 99 Gew.-% und mit Sauerstoff beladenen Fluorcarbonen im Bereich von 0,2 bis 100 % (Gewicht/Volumen) bestehen gemäß der DE-42 21 255, auf die Bezug genommen wird. Dabei haben die asymmetrischen lamellaren Aggregate eine Hautpenetrierung in Abhängigkeit von kritischen Löslichkeitstemperaturen der angewandten Fluorcarbone oder Fluorcarbongemische. Mit einer solchen Kombination wird ein Additionseffekt und teilweise ein synergistischer Effekt hinsichtlich der Sauerstoffversorgung der Haut erzielt. Mit Hilfe der asymmetrischen lamellaren Aggregate werden die hartmagnetischen Einbereichsteilchen in verkapselter Form in die Hautoberfläche eingebracht und üben vermutlich infolge ihrer Magnetkraft eine Sogwirkung auf die im Blut befindlichen Hämoglobinpartikel aus, die bis in die Spitzen der letzten Blutkapillaren "gezogen" werden. Dadurch wird eine höhere Versorgung der Haut mit Sauerstoff erreicht, was durch den von außen in die Haut hereingetragenen Sauerstoff mit Hilfe der asymmetrischen lamellaren Aggregate noch verstärkt wird.

Die für die asymmetrischen lamellaren Aggregate eingesetzten Phospholipide sind vorteilhaft ausgewählt aus der Gruppe, bestehend aus natürlichen Phospholipiden, wie Sojalecithin und Eilecithin, sowie den synthetischen Phospholipiden und/oder teilhydrierten Phospholipiden.

Besonders vorteilhaft ist es, wenn die verwendete Lipidfraktion Phophatidylcholin in sehr hohen Anteilen enthält, insbesondere in Anteilen von 70 bis 99 Gewichts-%. Neben Phosphatidylcholin können auch Lysolecithin im Konzentrationsbereich von 1 bis 10 Gewichts-% vorhanden sein.

Zur Erreichung einer langsamen Hautpenetrierung können Fluorcarbone oder Flourcarbongemische mit einer höheren kritischen Löslichkeitstemperatur enthalten sein.

Unter dem hier verwendeten Begriff "Fluorcarbone" werden perfluorierte oder hochfluorierte Kohlenstoffverbindungen oder Gemische verstanden, die in der Lage sind, Gase wie Sauerstoff und Kohlendioxid zu transportieren. Hochfluorierte Kohlenwasserstoffverbindungen sind im Sinne dieser Erfindung solche, bei denen die meisten Wasserstoffatome durch Fluoratome ersetzt sind, so daß bei weiterem Ersatz nicht notwendigerweise die Fähigkeit zum Gastransport erhöht wird. Dies wird meist dann erreicht, wenn etwa bis zu 90 % der Wasserstoffatome durch Fluoratome ersetzt sind. Bevorzugt im Sinne der vorliegenden Erfindung sind Fluoratome, bei denen wenigstens 95 % der Wasserstoffatome ersetzt sind, bevorzugter 98 % und am bevorzugtesten 100 %.

Es können eine Vielzahl von Fluorcarbonen eingesetzt werden, z.B. aliphatische geradkettige und verzweigte Fluoralkane, mono- oder bicyclische und gegebenenfalls fluoralkylsubstituierte Fluorcycloalkane, perfluorierte aliphatische oder dicyclische Amine, Bis-(perfluoralkyl)-Ethene, Perfluorpolyether oder deren Gemische. Besonders bevorzugt sind solche Fluorcarbone wie Perfluordecalin, F-Butyltetrahydrofuran, Perfluortributylamin, Perfluoroctylbromid, Bis-Fluor(butyl)-ethen oder Bis-Fluor(hexyl)ethen oder c₆-C₉-Perfluoralkane.

Wie bereits ausgeführt, können neben Phosphatidylcholin auch Lysolecithine eingesetzt werden und/oder geladene Phospholipide wie Phosphatidylethanolamin, n-Acetylphosphatidylethanolamin oder Phosphatidsäure im Konzentrationsbereich von 0,1 bis 30 Gewichts-%.

Gewünschtenfalls können die hartmagnetischen Einbereichsteilchen mit einer Schicht überzogen werden, die die Koerzitivfeldstärke nur wenig verringert, jedoch den Austritt von Barium- und/oder Strontiumionen verhindert oder hemmt. Dies kann dann erforderlich sein, wenn die Notwendigkeit besteht, nur solche Präparate einzusetzen, bei denen eine Auslaugung von Barium- oder Strontiumionen über einen bestimmten Zeitraum als Forderung der Gesundheitsbehörde vermieden werden soll. Hierfür kommen z.B. solche anorganischen Stoffe wie Titandioxid, Zirkondioxid oder Hydroxylapatit in Frage. Es können jedoch auch andere Stoffe eingesetzt werden, sofern sie die gleiche Funktion erfüllen, d.h. den Austritt von Barium- oder Strontiumionen durch Kochen mit Salzsäure über einen Zeitraum von dreißig Minuten unter dem zulässigen Wert zu halben.

Die Erfindung betrifft auch ein Verfahren zur Herstellung des Präparates. Das Verfahren besteht darin, daß die magnetischen Einbereichsteilchen gegebenenfalls unter Zusatz eines Dispergierhilfsmittels in einem üblichen Träger für pharmazeutische oder kosmetische Präparate und gegebenenfalls weiteren Additiven dispergiert werden mit Hilfe von Vorrichtungen mit Scherwirkung oder mit Ultraschallwirkung, z.B. bei Umdrehungszahlen im Bereich von 10 000 bis 27 000 U/min höher. Die Teilchengröße der hartmagnetischen Einbereichsteilchen liegt dabei im Bereich von 600 bis 1200 nm. Überraschenderweise erhält man dabei eine stabile Dispersion ohne die Bildung von normalerweise zu erwartenden Aggregaten und vermeidet damit ein Verklumpen des Endproduktes. Das ist für die kosmetische/dermatologische Anwendung wichtig, für die parenterale, z.B. intravenöse Verabreichung ist es zwingend, um ein stabiles kolloid-disperses System zu gewährleisten. Die für letztere Applikationsform notwendige Verarbeitung mit z.B. polymeren Stoffen wird erfindungsgemäß vermieden.

Für den Fall, daß die erfindungsgemäßen hartmagnetischen Einbereichsteilchen mit asymmetrischen lamellaren Aggregaten kombiniert werden sollen, erfolgt zuerst die Herstellung der asymmetrischen lamellaren Aggregate durch Voremulgierung von Fluorcarbonen in einer wäßrigen Phospholipidlösung bei etwa 12 000 bis 15 000 U/min. Daran schließt sich eine Hochdruckhomogenisierung zusammen mit den hartmagnetischen Einbereichsteilchen an, wobei entsprechende sphärische lamellare Strukturen entstehen. Zur Vermeidung von Autoxidationsprozessen im ungesättigten Säurerest nativer Lipide können Antioxidantien, z.B. α-Tocopherol zugesetzt werden. Der Fluorcarbongehalt und damit die Sauerstoffverfügbarkeit kann in breiten Grenzen variiert werden.

Die Erfindung betrifft auch die Verwendung eines Systems zur Herstellung eines pharmazeutischen oder kosmetischen Präparates zur Förderung der Durchblutung, indem eine Applikationsform für ein pharmazeutisches oder kosmetisches Präparat, bestehend aus einem Trägerstoff und gegebenenfalls weiteren Additiven und darin fein verteilten hartmagnetischen Einbereichsteilchen mit hoher Koerzitivfeldstärke und mit Korngrößen im Bereich von 600 bis 1200 nm in den Körper eingebracht oder auf die Haut aufgetragen wird. Dabei bestimmt die Menge der Einbereichsteilchen, die z.B. bei kosmetischer/dermaler Anwendung in die Haut eindringen und dort ein entsprechendes Magnetfeld aufbauen, die Wirkung hinsichtlich der Durchblutungsförderung. Die Ausnutzung der magnetischen Eigenschaften des Blutes zur Verbesserung der Blutzirkulation insbesondere in den feinen Kapillaren führt zu einer verbesserten Versorgung mit Sauerstoff, zu einer verbesserten Versorgung mit Nährstoffen und zu einem verbesserten Abtransport von Schlackestoffen. Dies führt zu einer Zurückbildung altersbedingter Falten der Haut, zu einer verbesserten Elastizität, zu einer Hautverjüngung und im Falle von Cellulite zu einem wesentlich verbesserten Krankheitsbild. Auch eine Haarwuchs-stimulierende Wirkung ist feststellbar.

Messungen unter physiologischen und konstanten Bedingungen zeigen, daß eine Steigerung der Mikrozirkulation von bis zu 200 % erreicht werden konnte. Unter Mikrozirkulation wird die Hautdurchblutung im Kapillargefäßbereich verstanden. Dieses Ergebnis beweist die Überlegenheit der erfindungsgemäßen Präparate gegenüber den bisherigen Ergebnissen des Standes der Technik.

Ein zusätzlicher Effekt ist erreichbar, wenn bei pharmazeutischen z.B. dermatologischen Präparaten gewünschte Arzneimittel in die Präparation einbezogen werden. Dies kann auf übliche Weise erfolgen, besonders vorteilhaft jedoch zum Beispiel dadurch, daß ein Einschluß dieser pharmazeutisch wirksamen Verbindungen zusammen mit den hartmagnetischen Einbereichsteilchen in die asymmetrischen lamellaren Aggregate erfolgt und damit ein tiefes Eindringen in die Haut gewährleistet wird.

Als pharmazeutisch wirksame Verbindungen kommen in Betracht: pharmakologische Wirkstoffe in Form von systemischen Wirkstoffen, einschließlich Cytostatika, Cancerostatika, Immunmodulatoren und Vakzinen, insbesondere solche der folgenden Gruppe: dermatologische Wirkstoffe, wie zum Beispiel Virustatika oder viruzide Arzneistoffe, Antimykotika, Heparine (z.B. Heparin-Calcium, Heparin-Natrium, niedermolekulare Heparine), Antibiotika, Corticoide, Antiinfektiosia, Aknewirkstoffe, Lokalanästhetika, Antiphlogistika, Antihistaminika oder Antipsoriatika; systemische Wirkstoffe, wie zum Beispiel nichtsteroidale Analgetika/Antirheumatika (z.B. Diclofenac-Natrium, Diclofenac-Diethylaminsalz, Etofenamat, Flufenaminsäure, 2-Hydroxyethylsalicylat, Ibuprofen, Indomethacin, Piroxicam), Opiatrezeptor-Agonisten und -Antagonisten (z.B. Buprenorphin, Fentanyl, Pentazocin, Pethidin, Tilidin, Tramadol, Naloxon), Histaminantagonisten (z.B. Bamipinlactat, Chlorphenoxamin-HCl, Clemastinhydrogenfumarat, Dimetindenmaleat, Pheniraminhydrogenmaleat), Insuline, regulatorische Peptide und ihre Hemmstoffe (z.B. Hypophysenvorderlappenhormone und ihre Hemmstoffe, Hypophyenhinterlappenhormone, Hypothalamushormone), Sedativa/ Hypnotika (z.B. Diazepam);
Wirkstoffe der Gruppe Cytostakika, Cancerostatika, Immunmodulatoren, Vakzine.

Ein bevorzugter dermatologischer Wirkstoff ist beispielsweise Rosmarinsäure oder ein anderer in Pflanzen vorkommender viruzider oder virustatischer Wirkstoff. Ein bevorzugter systemischer Wirkstoff ist beispielsweise ein niedermolekulares oder hochmolekulares Heparin, ein Oligopeptid oder ein Polypeptid.

Weitere bevorzugte Wirkstoffe sind Vitamine (E, A, B, C), Muramylpeptide, Doxorubicin, Gentamycin, Gramycidin, Dexamethason, Hydrocortison, Progesteron, Prednisolon bzw. davon abgeleitete Derivate und/oder Säure- bzw. Basenadditionssalze sowie Melanin.

Mit relevanten Wirkstoffen und Wirkstoffkombinationen wird bei entsprechenden Indikationen eine antineoplastische Therapie, eine antimikrobielle und antivirale Therapie sowie weitere Therapieart möglich, die infolge der verbesserten Sauerstoffversorgung der Haut mittels der erfindungsgemäßen Präparation auch zu einer verbesserten Aufnahme der pharmazeutischen Wirkstoffe führt und damit erfolgreicher wird.

Im allgemeinen sind die Wirkstoffmengen in therapeutischer Hinsicht sehr gering, so daß z.B. für den Fall löslicher Wirkstoffe Löslichkeiten von 0,5 bis 12 g/100 ml ausreichend für eine medizinische Anwendung sind. Sollten diese Löslichkeiten nicht gegeben sein, so ist auch die Emulgierung über das Zusammenwirken von z.B. Fluorcarbon und Phospholipid unter Anwendung bekannter Verfahren möglich, um zu der entsprechenden galenischen Zusammensetzung zu gelangen. Daher sind die Wirkstoffe in der aus bisheriger medizinischer Sicht ausreichenden Menge in den neuen Träger einarbeitbar.

Als Träger für die hartmagnetischen Einbereichsteilchen in einem kosmetischen Präparat können die für Seifen, Cremes, Lotionen, Emulsionen, Wässer, Auszüge, Pasten, Gele, Puder, Tinkturen üblichen Stoffe eingesetzt werden, wobei diese gegebenenfalls auch in Form eines Verbandes, eines Pflasters oder als Spray vorliegen können.

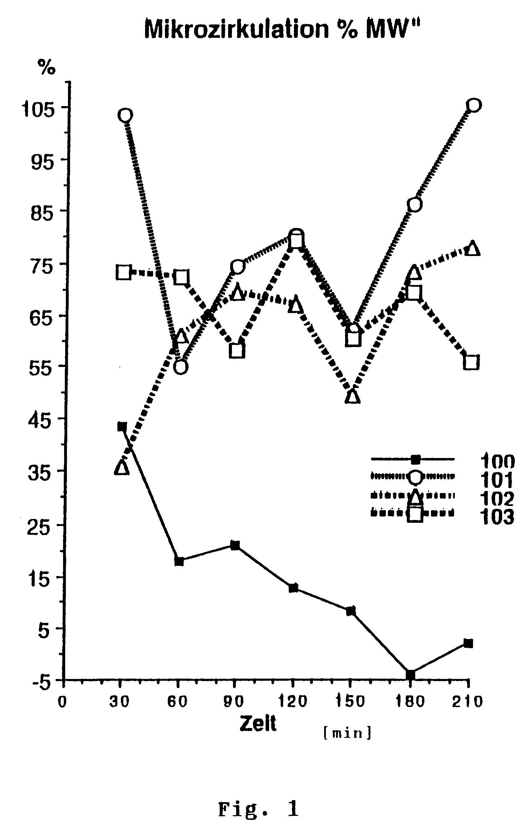

Die Erfindung soll nachstehend durch Beispiele näher erläutert werden. Die auf die kosmetische Anwendung bezogenen Beschreibungsabschnitte und Beispiele sollen jedoch keine Einschränkung der Erfindung darstellen. In der dazugehörigen Zeichnung bedeutet
Fig. 1 grafische Darstellung der Mikrozirkulation über die Zeit bei verschiedenen Testproben

### Beispiel 1

### Herstellung einer Suspension mit hartmagnetischen Pulvern aus Strontiumhexaferrit.

Einer Mischung aus Propylenglykol, Glycerin, destilliertes Wasser im Mischverhältnis 1 : 1 : 2 werden 5 Gewichtsprozente hartmagnetische Pulver aus Strontiumhexaferrit im Dickenverhältnis 5 : 1 vom Kornbereich 700 - 1000 nm zugegeben und homogenisiert. Dies erfolgt mit einem Turraxhomogenisator: 15000 U/min über eine Zeit von 30 min.

### Beispiel 2

### Herstellung einer Suspension mit hartmagnetischen Pulvern aus Bariumhexaferrit.

Einer Mischung aus Propylenglykol und destilliertem Wasser im Mischverhältnis 1 : 1 werden 15 Gewichts-% hartmagnetische Pulver aus Bariumhexaferrit im Dickenverhältnis 10 : 1 vom Kornbereich 600 - 800 nm zugegeben und homogenisiert.

| | |
|---|---|
| Ultraschalldesingrator: | 400 W |
| Amplitude: | 50 |
| Zeit: | 40 min. |

### Beispiel 3

### Herstellung einer Suspension mit hartmagnetischen Pulvern aus Bariumhexaferrit und Strontiumhexaferrit.

Einer Mischung aus Propylenglykol und destilliertem Wasser im Mischverhältnis 1 : 1 werden 30 Gewichts-% Bariumhexaferrit und Strontiumhexaferrit im Verhältnis 1 : 1 zugegeben. Das Dickenverhältnis beträgt beim Strontiumferrit 4 : 1 und beim Bariumferrit 5 : 1. Das Kornspektrum toleriert zwischen 700 und 1000 nm.

### Homogenisierungsparameter:

| | |
|---|---|
| Ultraschalldesingrator: | 400 W |
| Amplitude: | 50 |
| Zeit: | 45 min. |

### Beispiel 4.1

### Herstellung von Liposomen mit hartmagnetischen Pulvern aus Bariumhexaferrit und Strontiumhexaferrit.

In 29 Gewichts-% synthetisches Phosphorlipid und 1 Gewichts-% Lysolecithine werden 0,8 Gewichts-% hartmagnetisches Pulver aus Bariumhexaferrit im Dickenverhältnis 6 : 1 Kornbereich 600 - 800 nm dispergiert.

| | |
|---|---|
| Turraxhomogenisator: | 20000 U/min |
| Zeit: | 7 min |

Es ist als Austausch: 0,8 Gewichtsprozent Strontiumhexaferrit bzw. die Mischung Bariumhexaferrit:Strontiumhexaferrit im Verhältnis x % zu (100-x) unter gleichen technologischen Bedingungen möglich. Im Anschluß werden 10 % Ethanol und q.s. dest. Wasser zugegeben.

| | |
|---|---|
| Turraxhomogenisator: | 15000 U/min |
| Zeit: | 20 min |

### Beispiel 4.2

### Herstellung von Liposomen mit hartmagnetischen Pulvern aus Bariumhexaferrit und/oder Strontiumhexaferrit.

In 20 Gewichts-% teilhydrierten Phospholipiden, synthetischen Phospholipiden im Mischungsverhältnis 1 : 1 und 10 Gewichts-% Lysolecithine werden 70 Gewichts-% hartmagnetisches Pulver aus Bariumhexaferrit im Dickenverhältnis 7 : 1, Kornbereich 800 - 1000 nm dispergiert.

| | |
|---|---|
| Ultraschalldesintegrator: | 400 W |
| Amplitude: | 50 |
| Zeit: | 30 min |

Es ist als Austausch: 70 Gewichts-% Strontiumhexaferrit bzw. die Mischung Bariumhexaferrit:Strontiumhexaferrit im Verhältnis x % zu (100-x) unter gleichen technologischen Bedingungen möglich. Im Anschluß werden 10 % Ethanol und q. s. dest. Wasser zugegeben.

| | |
|---|---|
| Ultraschalldesintegrator: | 400 W |
| Amplitude: | 50 |
| Zeit: | 70 min |

### Beispiel 5

### Herstellung von asymmetrischen lamellaren Aggregaten mit hartmagnetischen Pulvern aus Bariumhexaferrit und/oder Strontiumhexaferrit.

In 8 Gewichts-% Phosphorlipiden mit einem Phosphatidylcholingehalt von 30 Gewichts-% Eilecithin werden 0,01 Gewichts-% hartmagnetisches Pulver aus Bariumhexaferrit im Dickenverhältnis 3 : 1, Kornbereich 750 - 900 nm dispergiert.

| | |
|---|---|
| Turraxhomogenisator: | 27000 U/min |
| Zeit: | 5 min |

Es ist als Austausch: 0,01 Gewichts-% Strontiumhexaferrit bzw. die Mischung Bariumhexaferrit:Strontiumhexaferrit im Verhältnis x % zu (100-x) unter gleichen technologischen Bedingungen möglich. Im Anschluß folgt die Zugabe von 6,0 Gewichts-% Glycerin, 6 Gewichts-% Propylenglykol, 0,2 Gewichts-% mit Sauerstoff beladenen Fluorcarbone und q.s. destilliertes Wasser.

| | |
|---|---|
| Turraxhomogenisator: | 25000 U/min |
| Zeit: | 20 min |

### Beispiel 6

### Herstellung von asymmetrischen lamellaren Aggregaten mit hartmagnetischen Pulvern aus Bariumhexaferrit und/oder Strontiumhexaferrit.

In 10 Gewichts-% Phosphorlipiden von 99 Gewichts-% Phosphatidylcholingehalt, Sojalecithin, werden 1,0 Gewichts-% hartmagnetisches Pulver aus Bariumhexaferrit im Dickenverhältnis 10 : 1, Kornbereich 800 - 950 nm dispergiert.

| | |
|---|---|
| Turraxhomogenisator: | 27000 U/min |
| Zeit: | 10 min |

Es ist als Austausch: 1,0 Gewichtsprozent Strontiumhexaferrit bzw. die Mischung Bariumhexaferrit:Strontiumhexaferrit im Verhältnis x % zu (100-x) unter gleichen technologischen Bedingungen möglich. Im Anschluß folgt die Zugabe von 6,0 Gewichts-% Glycerin, 6 Gewichts-% Propylenglykol, 50 Gewichts-% mit Sauerstoff beladenen Fluorcarbone und q.s. destilliertes Wasser.

| | |
|---|---|
| Turraxhomogenisator: | 27000 U/min |
| Zeit: | 20 min |

### Beispiel 7

### Herstellung von Fluorcarbondispersion mit hartmagnetischen Pulvern aus Bariumhexaferrit und/oder Strontiumhexaferrit.

In 100 Gewichts-% sauerstoffbeladene Fluorcarbone werden 4,0 Gewichts-% hartmagnetisches Pulver aus Bariumhexaferrit im Dickenverhältnis 4 : 1, Kornbereich 850 - 1000 nm dispergiert.

| | |
|---|---|
| Ultraschalldesintegrator: | 400 W |
| Amplitude: | 50 |
| Zeit: | 25 min |

Es ist als Austausch: 0,4 Gewichtsprozent Strontiumhexaferrit bzw. die Mischung Bariumhexaferrit:Strontiumhexaferrit im Verhältnis x % zu (100-x) unter gleichen technologischen Bedingungen möglich.

### Beispiel 8

### Herstellung von Fluorcarbondispersion mit hartmagnetischen Pulvern aus Bariumhexaferrit und/oder Strontiumhexaferrit.

In 100 Gewichtsprozenten sauerstoffbeladene Fluorcarbone werden 60 Gewichts-% hartmagnetisches Pulver aus Strontiumhexaferrit im Dickenverhältnis 9 : 1, Kornbereich 900 - 1200 nm dispergiert.

| | |
|---|---|
| Ultraschalldesintegrator: | 400 W |
| Amplitude: | 50 |
| Zeit: | 60 min |

Es ist als Austausch 60 Gewichts-% Bariumhexaferrit bzw. die Mischung Bariumhexaferrit:Strontiumhexaferrit im Verhältnis x % zu (100-x) unter gleichen technologischen Bedingungen möglich.

### Beispiel 9 Dermatologische Salbe

### Phase A:

| | |
|---|---|
| Bienenwachs | 8 % |
| Lanolin synthetisch | 10 % |

### Phase B:

| | |
|---|---|
| Glycerin | 10 % |
| dest. H₂O | qs |

### Phase C:

| | |
|---|---|
| Fluorcarbondispersion nach Beispiel 7 | 50 % |

### Herstellung:

Phase A wird unter Rühren aus 65 °C erhitzt. Phase B wird ebenfalls auf 65 °C erwärmt und bei Temperaturstabilität der Phase A unter Rühren zugegeben.Die Homogenisierungszeit beträgt 10 Minuten. Danach erfolgt die Abkühlungsphase. Ist die Temperatur von ≤ 30 °C ereicht, wird unter langsamen Rühren Phase C zugegeben.

### Beispiel 10 Dermatologische Paste

### Phase A:

| | |
|---|---|
| Cetyl Stearylalkohol | 5 % |
| Bienenwachs | 15 % |
| Lanolin synthetisch | 20 % |

### Phase B:

| | |
|---|---|
| Propylenglykol | 5 % |
| Glycerin | 5 % |
| dest. H₂O | qs |

### Phase C:

| | |
|---|---|
| Fluorcarbondispersion nach Beispiel 6 | 30 % |

### Herstellung:

Phase A wird unter Rühren auf 65 °C erhitzt. Phase B wird ebenfalls auf 65 °C erwärmt und bei Temperaturstabilität der Phase A unter Rühren zugegeben. Die Homogenisierungszeit beträgt 10 Minuten. Danach erfolgt die Abkühlungsphase. Ist die Temperatur von ≤ 30 °C erreicht, wird unter langsamen Rühren Phase C zugegeben.

### Beispiel 11 Dermatologische Paste

| | |
|---|---|
| Glycerin | 10 % |
| Propylenglykol | 5 % |
| Fluorcarbondispersion | 85 % |

Bei Raumtemperatur werden die Rohstoffe nacheinander vermischt.

### Beispiel 12 Dermatologische Tinktur

| | |
|---|---|
| Glycerin | 5 % |
| Propylenglykol | 5 % |
| Wasser | q.s. |
| Suspension mit hartmagn. Pulver nach Beispiel 1 | 1 % |

Alle Rohstoffe werden bei Raumtemperatur in Wasser vermischt. Die Reihenfolge kann beliebig gewählt werden.

### Beispiel 13 W/O Emulsion

| | |
|---|---|
| Emulgatorsystem | 8,2 % |
| bestehend aus: - Phosphorsäureester, Isopropylpalmitat Verhältnis 35 % : 65 % | |
| Paraffin | 12,2 % |
| Glycerin | 5,3 % |
| Konservierungsmittel | 0,3 % |
| Wasser | q.s. |
| Dispersion mit hartmagn. Pulver nach Beispiel 5 | 10,0 % |

Kaltherstellung : Die Rohstoffe werden der Reihe nach vermischt und anschließend ca. 10 Minuten homogenisiert.

### Beispiel 14 O/W Emulsion

### Phase A:

| | |
|---|---|
| Glycerylstearat | 1,0 % |
| Stearinsäure | 2,0 % |
| Kakaobutter | 3,0 % |
| Cetylalkohol | 1,5 % |
| Oleylalkohol | 0,5 % |
| Dimethiocon | 1,0 % |
| Dinatrium-EDTA | 0,15 % |
| Butylacetat-Hydroxytoluen | 0,05 % |

### Phase B:

| | |
|---|---|
| dest. H₂O | qs |
| Carbomer | 0,5 |
| Propylenglykol | 3,5 % |
| Glycerin | 2,5 % |
| Konservierungsmittel | 0,5 % |

### Phase C

| | |
|---|---|
| TEA | 0,5 % |

### Phase D

| | |
|---|---|
| Parfümöl | 0,5 % |
| Dispersion mit hartmagn. Pulver gemäß Beispiel 5 | 5,0 % |

Herstellung: Phase A wird unter Rühren auf 80 °C erhitzt.
Phase B wird ebenfalls auf 80 °C erwärmt.

### Beispiel 15 Kosmetisches Gel

| | |
|---|---|
| dest. Wasser | q.s. % |
| Carbomer | 0,6 % |
| TEA | 0,6 % |
| Konservierungsmittel | 0,3 % |
| Propylenglykol | 3,5 % |
| Glycerin | 4,0 % |
| Naturöl | 2,0 % |
| Parfümöl | 0,5 % |
| Suspension mit hartmagn. Pulver gemäß Beispiel 2 | 2,5 % |

Herstellung/Kaltherstellung: Wasser und Carbomer werden bei Raumtemperatur homogenisiert. Die Zugabe der restlichen Rohstoffe erfolgt in Reihenfolge unter Rühren.

### Beispiel 16 Lotion

| | |
|---|---|
| Polyacrylsäure Mol 4 Mill | 0,5 % |
| Truthanolamin | 0,5 % |
| Cetyl-Stearylalkohol | 2,0 % |
| Propylglykol | 2,0 % |
| Glycerin | 1,5 % |
| Vitamin E | 1,0 % |
| dest. Wasser | q.s. |
| Parfümöl | 0,5 % |
| Konservierungsmittel | 0,3 % |
| Dispersion mit hartmagn. Pulver nach Beispiel 5 | 3,5 % |

Die Herstellung/Kaltherstellung erfolgt nach Beispiel 14.

### Beispiel 17 Haarwasser

| | |
|---|---|
| dest. Wasser | q.s. |
| Carbomer | 0,05 % |
| TEA | 0,1 % |
| Vitamin B | 1,0 % |
| Propylenglykol | 2,0 % |
| Parfümöl | 0,5 % |
| Suspension mit hartmagn. Pulver nach Beispiel 3 | 1,5 % |

Die Herstellung/Kaltherstellung erfolgt nach Beispiel 15.

### Beispiel 18 Haar-Kopfhautpackung

| | |
|---|---|
| dest. Wasser | q.s. |
| Cetylalkohol | 3,0 % |
| Phosphorsäureester | 6,5 % |
| Isopropylamid 1 : 1 | |
| Cocolglyceride O | 3,5 % |
| Stearinsäure | 6,0 % |
| Glycerin | 5,0 % |
| Lecithin | 1,0 % |
| Liposome nach Beispiel 4.1 | 20,0 % |

Die Herstellung/Kaltherstellung erfolgt nach Beispiel 15

### Beispiel 19 O/W Spezialemulsion

### Grundlage

### Phase A

| | |
|---|---|
| Cetearylalkohol | 1,5 % |
| Cetearylalkohol und PEG-40 Castor Oil | 3,0 % |
| Verhältnis 1 : 1 | |
| Hexyllaurat | 1,5 % |
| Dibutyladipat | 4,0 % |
| Oleylerucat | 1,5 % |

### Phase B

| | |
|---|---|
| Dest. Wasser | q.s. |
| Carbomer | 0,3 % |
| Allantoin | 0,2 % |

### Phase C

| | |
|---|---|
| TEA | 0,3 % |

### Phase D

| | |
|---|---|
| Aloe Vera | 2,0 % |
| Siliconoil | 3,0 % |
| D-Panthenol | 0,5 % |
| Babassuoil | 2,0 % |
| Vitamin A Palmitat | 1,0 % |
| Olivenoil | 2,0 % |
| Konservierungsmittel | 0,3 % |
| Asymmetrische lamellare Aggregate mit hartmagnetischen Pulvern | 15,0 % |

Die Herstellung erfolgt gemäß Beispiel 14.

### Beispiel 20

| | |
|---|---|
| Aloe Vera Gel | 10,0 % |
| Algengel | 5,0 % |
| Ethanol | 10,0 % |
| dest. Wasser | q.s. |
| magn. Pulver nach Beispiel 1 | 55,0 % |

Die Herstellung erfolgt nach Beispiel 11

### Beispiel 21 Beinserum

### Phase A

| | |
|---|---|
| Cetearyl Alkohol | 3,5 % |
| Cetearyl Alkohol | 1,0 % |

### Phase B

| | |
|---|---|
| Carbomer | 0,5 % |
| Dest. Wasser | q.s. |
| Konservierungsmittel | 0,3 % |

### Phase C

| | |
|---|---|
| TEA | 0,5 % |

### Phase D

| | |
|---|---|
| Aloe Vera | 1,5 % |
| Liposome mit hartmagnetischen Pulvern nach Ausführungsbeispiel 6 | 30,0 % |

Die Herstellung erfolgt nach Beispiel 14.

### Beispiel 22 Shampoo

### Phase A

| | |
|---|---|
| Natriumlaurylethersulfat | 38,0 % |
| Monoethanolammoniumlaurylsulfat | 10,0 % |
| Octamethylcyclotetrasiloxan | 5,0 % |
| Jojobaöl | 0,5 % |
| dest. Wasser | q.s. |
| Konservierungsmittel | 0,3 % |
| Fette | 0,01 % |
| Parfümöl | 0,5 % |
| Suspension mit hartmagnetischen Pulvern nach Beispiel 2 | 3,5 % |

Zur Herstellung werden die Rohstoffe im Wasser bei Raumtemperatur vermischt.

### Beispiel 23 Dekoratives Puder

| | |
|---|---|
| Talcum | q.s. |
| Kaolin | 9,5 % |
| Magnesiumstearat | 2,5 % |
| Magnesiumcarbonat | 2,5 % |
| Zinkstearat | 1,5 % |
| Farbenkombination | 3,5 % |
| je nach Farbton Suspension mit hartmagnetischen Pulvern | 5,0 % |

Zu Herstellung werden die Rohstoffe der Reihe nach homogen zusammengemischt.

### Beispiel 24 Dusch-Cremebad

| | |
|---|---|
| Natriumlaurylethersulfat | 35,0 % |
| Glycerylstearat und Ceteareth-20 Verhältnis 1 : 1 | 2,0 % |
| Glycerylisostearat | 3,0 % |
| Jojobaöl | 1,0 % |
| Konservierungsmittel | 0,3 % |
| Parfümöl | 0,3 % |
| Suspension mit hartmagnetischen Pulvern nach Beispiel 1 | 1,5 % |

Zu Herstellung werden die Rohstoffe bei Raumtemperatur vermischt.

### Beispiel 25 Make-up flüssig

| | |
|---|---|
| Emulgatorensystem | 6,5 % |
| bestehend aus: Glycerylstearat, Ceteareth-20, Ceteareth-12, Cetearylalkohol, Cetylpalmitat in annähernd gleichen Verhältnissen | |
| Glycerin | 2,5 % |
| Propylenglycol | 1,5 % |
| Aloe Vera Extrakt | 0,5 % |
| Vitamin E | 1,0 % |
| Farben | 3,5 % |
| Dispersion nach Beispiel 7 mit hartmagn. Pulvern | 10,5 % |
| Wasser | q.s. |

Die Herstellung entspricht Beispiel 14.

### Beispiel 26 Lippenstift

| | |
|---|---|
| Rizinusöl | q.s. |
| Bienenwachs | 13,0 % |
| Carnaubawachs | 8,5 % |
| Lanolin | 5,0 % |
| Paraffin | 3,0 % |
| Konservierungsmittel | 0,05 % |
| Perlglanzpigmente | 5,0 % |
| Dispersion mit hartmagnetischen Pulvern nach Beispiel 8 | 1,0 % |
| Farbpigmente | 3,0 % |

Herstellung: Unter intensivem Rühren wird die Schmelze auf 80 °C erhitzt. Die Zugabe der Pigmente erfolgt bei 60 °C. Die Gießtemperatur liegt bei 60 °C.

### Beispiel 27 Gesichtsmaske

| | |
|---|---|
| Emulgatorensystem | 9,5 % |
| bestehend aus: Polyglycerinester Verhältnis 2:1 Stabilisatoren | |
| Paraffinum | 12,0 % |
| Glycerin | 5,3 % |
| Talcum | 2,0 % |
| Ton | 1,0 % |
| Konservierungsmittel | 0,3 % |
| dest. Wasser | q.s. |
| Dispersion mit hartmagnetischen Pulvern nach Beispiel 8 | 30 % |

Zur Herstellung werden die Rohstoffe bei Raumtemperatur der Reihe nach zugegeben und homogen vermischt.

### Beispiel 28 Sonnenprodukt

| | |
|---|---|
| Emulgatorensystem | 10,5 %, |
| bestehend aus: Phosphorsäureester Verhältnis 1:1 Isopropylpalmitat | |
| Palmitinsäureisopropylester | 1,5 % |
| Vaseline | 5,5 % |
| Paraffinum | 5,0 % |
| MgSO₂ · 7H₂O | 0,5 % |
| Glycerin | 1,5 % |
| Talcum | 2,0 % |
| Konservierungsmittel | 0,5 % |
| UV-Filter | 6,0 % |
| TiO₂ | 3,0 % |
| dest. Wasser | q.s. |

Dispersion mit hartmagn. Pulvern nach Beispiel 7
Zur Herstellung werden die Rohstoffe bei Raumtemperatur der Reihe nach zugegeben und homogen vermischt.

### Beispiel 29 Pharmazeutisches Puder

| | |
|---|---|
| Talcum | q.s. |
| Kaolin | 15,5 % |
| Magnesiumstearat | 5,0 % |
| Zinkoxid | 2,0 % |
| Magnesiumcarbonat | 2,0 % |
| Suspension mit hartmagn. Pulvern nach Beispiel 1. | 1,5 % |

Zur Herstellung werden die Rohstoffe der Reihe nach homogen zusammengemischt.

### Beispiel 30

Gemäß Beispiel 19 hergestellte kosmetische Präparate wurden einem Anwendungstest unterzogen, bei dem die Mikrozirkulation der Haut nach Auftragen einer salbenförmigen Probe gemessen wurde.

Die Hautdurchblutung ergibt sich bekanntlich aus dem Produkt Blutfluß mal Gefäßvolumen. Die Kapillargefäße unterliegen neben der Vasodilatation und Konstriktion einer pulsierenden Vasomotion, die als Kapillarpuls bezeichnet wird. Zur quantitativen Bestimmung der Mikrozirkulation wurde die Laser-Doppler-Flußmessung mit einem Periflux-Gerät (Perimet KB, Schweden) eingesetzt. Der 2 mW Helium-Neon-Laser wurde über einen flexiblen Lichtleiter an die Meßstelle geleitet. Die Faseroptik wurde durch eine Halterung auf den Meßbereich fixiert, wobei eine Eindringtiefe des Laserlichtes in das Hautgewebe von 1,5-2 mm gewährleistet war. Gemessen wurde eine Spannung als Aus- und Eingangssignal, das als relatives Maß der Gewebedurchblutung direkt proportional dem Produkt aus Erythrozytenmenge und Erythrozytengeschwindigkeit ist. Mit dem Verfahren war eine kontinuierliche, berührungsfreie und quantitative Registrierung der Hautdurchblutung möglich. Da die Hauttemperatur einen großen Einfluß auf die kutane Mikrozirkulation beziehungsweise auf die Reaktionsfähigkeit der Kapillargefäße hat, mußten die Umgebungsbedingungen den physiologisch indifferenten Bereichen genähert und über den gesamten Versuchsdurchlauf konstant gehalten werden. Diese Versuchsbedingungen betrugen für Versuchspersonen 26° c ± 1 und 36 % ± 1 relative Feuchte wobei die Versuchspersonen bereits dreißig Minuten vorher an diese Bedingungen angepaßt wurden. Nach einer Nullmessung ließ man die Salbenproben im Überschuß dreißig Minuten auf eine Hautfläche an der Unterarm-Innenseite einwirken. Das Signifikanzniveau betrug p < 0,05.

Bei den Versuchspersonen, die auf die aufgetragene Salbe reagierten, wurde eine Steigerung der Mikrozirkulation von bis zu 200 % festgestellt. Gemäß Fig.1 zeigt sich die über dem Ausgangswert M100 eine deutliche Erhöhung der Mikrozirkulation bei Proben mit Anteilen an hartmagnetischen Einbereichsteilchen, hier Probe M101.

## Patentansprüche

1. Präparat zur Durchblutungsförderung, gekennzeichnet durch pharmazeutisch oder kosmetisch annehmbare Trägerstoffe und/oder Additive und darin fein verteilte hartmagnetische Einbereichsteilchen mit hoher Koerzitivfeldstärke und mit Korngrößen im Bereich von 600 bis 1200 nm.

2. Präparat nach Anspruch 1, dadurch gekennzeichnet, daß die hartmagnetischen Einbereichsteilchen Barium- und/oder Strontiumhexaferrite sind, vorzugsweise undotierte Barium und/oder Strontiumhexaferrite.

3. Präparat nach Anspruch 2, dadurch gekennzeichnet, daß die hartmagnetischen Einbereichsteilchen aus Bariumhexaferrit bestehen.

4. Präparat nach Anspruch 2 oder 3, dadurch gekennzeichnet, daß die hartmagnetischen Einbereichsteilchen mit hoher Koerzitivfeldstärke Barium- und/oder Strontiumhexaferrite sind, erzeugt nach der Glaskristallisationstechnik durch Züchtung von Einkristallen aus einer abgeschreckten Glasschmelze.

5. Präparat nach einem der Ansprüche 1 bis 4, dadurch gekennzeichnet, daß die Teilchengröße der Einbereichsteilchen in einem engen Bereich liegt, vorzugsweise im Bereich von 750 bis 1000 nm, insbesondere 800 bis 950 nm.

6. Präparat nach einem der Ansprüche 1 bis 5, dadurch gekennzeichnet, daß die hartmagnetischen Einbereichsteilchen mit einer Schicht überzogen sind, die die Koerzitivfeldstärke nicht oder wenig verringert und die den Austritt von Barium- und/oder Strontiumionen verhindert oder hemmt.

7. Präparat nach einem der Ansprüche 1 bis 6, dadurch gekennzeichnet, daß die hartmagnetischen Einbereichsteilchen zur kosmetischen und dermatologischen Anwendung in Kombination vorliegen mit asymmetrischen lamellaren Aggregaten, die aus Phospholipiden mit einem Phosphatidylcholingehalt im Bereich von 30 bis 99 Gew.-% und mit Sauerstoff beladenen Fluorcarbonen im Bereich von 0,2 bis 100 % (Gewicht/Volumen) bestehen, wobei die asymmetrischen lamellaren Aggregate eine Hautpenetrierung in Abhängigkeit von der kritischen Löslichkeitstemperatur der angewandten Fluorcarbone oder Fluorcarbongemische haben.

8. Präparat nach Anspruch 7, dadurch gekennzeichnet, daß die Phospholipide ausgewählt sind aus der Gruppe, bestehend aus natürlichen Phospholipiden wie Sojalecithin und Eilecithin sowie synthetischen Phospholipiden und/oder teilhydrierten Phospholipiden.

9. Präparat nach Anspruch 7 oder 8, dadurch gekennzeichnet, daß neben Phosphatidylcholin Lysolecithine im Konzentrationsbereich von 1 bis 10 Gew.-% vorhanden sind.

10. Präparat nach einem der Ansprüche 1 bis 9, dadurch gekennzeichnet, daß es zusätzlich pharmazeutisch wirksame Verbindungen enthält, ausgewählt unter Cytostatika, Cancerostatika, Immunmodulatoren, Vakzinen, Virustatika, viruziden Arzneistoffen, Antimykotika, Heparinen, Antibiotika, Corticoiden, Antiinfektiosia, Aknewirkstoffen, Lokalanästhetika, Antiphlogistika, Antihistaminika, Antipsoriatika, nichtsteroidalen Analgetika, nichtsteroidalen Antirheumatika, Opiatrezeptor-Agonisten und -Antagonisten, Histaminantagonisten, Insulinen, regulatorischen Peptiden und ihren Hemmstoffe, Sedativa, Hypnotika, Rosmarinsäure oder anderen in Pflanzen vorkommenden viruziden oder virustatischen Wirkstoffen, Vitaminen (E, A, B, C), Oligopeptiden oder Polypeptiden, oder das Pigment Melanin enthält.

11. Verfahren zur Herstellung eines Präparates zur Durchblutungsförderung, dadurch gekennzeichnet, daß in pharmazeutisch oder kosmetisch annehmbare Trägerstoffe und/oder Additive hartmagnetische Einbereichsteilchen mit hoher Koerzitivfeldstärke und mit Korngrößen im Bereich von 600 bis 1200 nm durch Dispergierung eingearbeitet werden, gegebenenfalls unter Zusatz von für die Anwendung üblichen Dispergierhilfsmitteln.

12. Verfahren nach Anspruch 11, dadurch gekennzeichnet, daß eine kosmetische oder dermatologische Formulierung hergestellt wird mit Anteilen der hartmagnetischen Einbereichsteilchen im Bereich von 0,01-70 Gew.-%, bezogen auf die Gesamtmasse der Dispersion.

13. Verfahren nach Anspruch 12 zur Herstellung eines kosmetischen oder dermatologischen Präparates, dadurch gekennzeichnet, daß die hartmagnetischen Einbereichsteilchen in Kombination mit asymmetrischen lamellaren Aggregaten, die aus Phospholipiden mit einem Phosphatidylcholingehalt im Bereich von 30 bis 99 Gew.-% und mit Sauerstoff beladenen Fluorcarbonen im Bereich von 0,2 bis 100 % (Gewicht/Volumen) bestehen, in den Trägerstoff eingebracht werden.

14. Verwendung eines Systems zur Herstellung eines Präparates zur Durchblutungsförderung, wobei das System einen pharmazeutisch annehmbaren Trägerstoff und darin dispergierte hartmagnetische Einbereichsteilchen mit hoher Koerzitivfeldstärke und mit Korngrößen im Bereich von 600 bis 1200 nm umfaßt.

15. Verwendung eines Systems zur Herstellung eines kosmetischen oder dermatologischen Präparates zur Durchblutungsförderung zum Auftragen auf die Haut, wobei das Präparat hartmagnetische Einbereichsteilchen mit hoher Koerzitivfeldstärke und mit Korngrößen im Bereich von 600 bis 1200 nm enthält, die in kosmetisch oder dermatologisch annehmbaren Trägerstoffen und gegebenenfalls Additiven dispergiert sind und wobei das System in einem für die kosmetische oder dermatologische Anwendung üblichen Träger wie Salben, Cremes, Lotionen, Wässer, alkoholische Auszüge, Pasten, Gele, Puder, Tinkturen verteilt oder gegebenenfalls auf einem Verband oder einem Pflaster oder als Spray vorliegt.

16. Verwendung nach Anspruch 14 oder 15, wobei das Präparat neben den hartmagnetischen Einbereichsteilchen mit hoher Koerzitivfeldstärke und mit Korngrößen im Bereich von 600 bis 1200 nm zur gleichzeitigen Steuerung der Sauerstoffversorgung der Haut als asymmetrische lamellare Sauerstoff-Carrier Phospholipide mit einem Phosphatidylcholingehalt von 30 bis 99 Gew.% und Fluorcarbone im Bereich von 0,2 bis 100 % Gewicht/Volumen umfaßt, und wobei die Penetration in die Haut über die Carrier-Struktur der Phopholipid-Aggregate und die kritische Löslichkeitstemperatur der Fluorcarbone (in n-Hexan) gesteuert wird.

17. Verwendung nach Anspruch 14 bis 16, dadurch gekennzeichnet, daß ein System nach Anspruch 1 oder 7 zusätzlich pharmazeutisch wirksame Substanzen enthält.

## Claims

1. A preparation for stimulating the circulation, characterized by pharmaceutically or cosmetically acceptable excipients and/or additives, in which there are finely divided, magnetically hard single-domain particles with a strong coercive field and with sizes in the range 600 to 1200 nm.

2. A preparation according to Claim 1, characterized in that the magnetically hard single-domain particles are barium and/or strontium hexaferrite, preferably undoped barium and/or strontium hexaferrite.

3. A preparation according to Claim 2, characterized in that the magnetically hard single-domain particles consist of barium hexaferrite.

4. A preparation according to Claim 2 or 3, characterized in that the magnetically hard single-domain particles with a strong coercive field are barium and/or strontium hexaferrites produced by growing single crystals from a tempered glass melt in accordance with the glass crystallization technique.

5. A preparation according to one of Claims 1 to 4, characterized in that the size of the single-domain particles is in a narrow range, preferably in the range 750 to 1000 nm and especially 800 to 950 nm.

6. A preparation according to one of Claims 1 to 5, characterized in that the magnetically hard single-domain particles are coated with a layer which reduces the coercive field only slightly, if at all, and prevents or inhibits the escape of barium and/or strontium ions.

7. A preparation according to one of Claims 1 to 6, characterized in that the magnetically hard single-domain particles for cosmetic and dermatological application are present in combination with asymmetric lamellar aggregates consisting of phospholipids with a phosphatidylcholine content in the range 30 to 99% by weight and oxygen-charged fluorocarbons in the range 0.2 to 100% (weight/volume), the asymmetric lamellar aggregates penetrating the skin as a function of the critical solubility temperature of the fluorocarbons or fluorocarbon mixtures used.

8. A preparation according to Claim 7, characterized in that the phospholipids are selected from the group consisting of natural phospholipids, such as soya lecithin and egg lecithin, as well as synthetic phospholipids and/or partially hydrogenated phospholipids.

9. A preparation according to Claim 7 or 8, characterized in that, in addition to phosphatidylcholine, lysolecithins are present in the concentration range 1 to 10% by weight.

10. A preparation according to one of claims 1 to 9, characterized in that the preparation comprises additionally pharmaceutically active compounds selected from cytostatic agents, carcinostatic agents, immunomodulators, vaccines, virustatic agents, virucidal medicinal agents, antimycotic agents, heparins, antibiotics, corticoids, antiinfective agents, active ingredients for acne, local anaesthetics, antiphlogistics, antihistamines, antipsoriatics , non-steroidal analgesics, non-steroidal antirheumatics, opiate receptor agonists and antagonists, histamine antagonists, insulins, regulatory peptides and their inhibitors, sedatives, hypnotics, rosmaric acid or another virucidal or virustatic active ingredient occurring in plants, oligopeptides, polypeptides, vitamins (E, A, B, C) and also the pigment melanin.

11. A process for the manufacture of a preparation for stimulating the circulation, characterized in that magnetically hard single-domain particles with a strong coercive field and with sizes in the range 600 to 1200 nm are incorporated into pharmaceutically or cosmetically acceptable excipients and/or additives by dispersion, with the optional addition of dispersants conventionally used for this purpose.

12. A process according to Claim 11, characterized in that a cosmetic or dermatological formulation is prepared with proportions of magnetically hard single-domain particles in the range 0.01 to 70% by weight, based on the total mass of the dispersion.

13. A process according to Claim 12 for the manufacture of a cosmetic or dermatological preparation, characterized in that the magnetically hard single-domain particles are incorporated into the excipient in combination with asymmetric lamellar aggregates consisting of phospholipids with a phosphatidylcholine content in the range 30 to 99% by weight and oxygen-charged fluorocarbons in the range 0.2 to 100% (weight/volume).

14. Use of a preparation containing magnetically hard single-domain particles with a strong coercive field and with sizes in the range 600 to 1200 nm, dispersed in a pharmaceutically acceptable excipient, for stimulating the circulation.

15. Use of a system for preparing a cosmetic or dermatological preparation for stimulating the circulation by application to the skin of magnetically hard single-domain particles with a strong coercive field and with sizes in the range 600 to 1200 nm, which are dispersed in cosmetically or dermatologically acceptable excipients and optionally additives, the system being dispersed in an excipient conventionally used for cosmetic or dermatological application, such as ointments, creams, lotions, colognes, alcoholic extracts, pastes, gels, powders or tinctures, or, where appropriate, being on a dressing or plaster or in the form of a spray.

16. Use according to Claim 14 or 15 of a preparation for simultaneous control of the oxygen supply to the skin by application of a system with an asymmetric lamellar oxygen carrier, comprising phospholipids with a phosphatidylcholine content of 30 to 99% by weight and fluorocarbons in the range 0.2 to 100% by weight/volume, and magnetically hard single-domain particles with a strong coercive field and with sizes in the range 600 to 1200 nm, the skin penetration being controlled by the carrier structure of the phospholipid aggregates and the critical solubility temperature of the fluorocarbons (in n-hexane).

17. Use according to Claim 14 to 16 for the additional supply of pharmaceutically active compounds by application of a system according to Claim 1 or 7 which also contains pharmaceutically active substances.

## Revendications

1. Préparation favorisant l'irrigation sanguine, caractérisée par des substances vectrices et/ou des additifs compatibles sur le plan pharmaceutique ou cosmétique, et par des particules mono-orientées fortement magnétiques s'y trouvant finement distribuées, particules présentant une intensité de champ coercitif élevée et des granularités de l'ordre de 600 à 1200 nm.

2. Préparation suivant la revendication 1, caractérisée en ce que les particules mono-orientées fortement magnétiques sont des hexaferrites de baryum et/ou de strontium, de préférence des hexaferrites de baryum et/ou de strontium non dotées.

3. Préparation suivant la revendication 2, caractérisée en ce que les particules mono-orientées fortement magnétiques se composent d'hexaferrite de baryum.

4. Préparation suivant la revendication 2 ou 3, caractérisée en ce que les particules mono-orientées fortement magnétiques à intensité de champ coercitif élevée sont des hexaferrites de baryum et/ou de strontium, produites selon la technique de la cristallisation du verre, par culture de monocristaux à partir d'un bain de fusion de verre trempé.

5. Préparation suivant l'une des revendications 1 à 4, caractérisée en ce que la grosseur des particules mono-orientées se situe dans une plage étroite, de préférence dans la plage de 750 à 1000 nm, en particulier de 800 à 950 nm.

6. Préparation suivant l'une des revendications 1 à 5, caractérisée en ce que les particules mono-orientées fortement magnétiques sont revêtues d'une couche qui ne diminue pas, ou seulement peu, l'intensité du champ coercitif, et qui empêche ou ralentit la perte d'ions de baryum et/ou de strontium.

7. Préparation suivant l'une des revendications 1 à 6, caractérisée en ce que les particules mono-orientées fortement magnétiques destinées à une application cosmétique ou dermatologique se présentent en combinaison avec des agrégats lamellaires asymétriques se composant de phospholipides ayant une teneur en phosphatidylcholine de l'ordre de 30 à 99% en poids, et avec des fluorocarbones chargés en oxygène dans l'intervalle de 0,2 à 100% (poids/volume), les agrégats lamellaires asymétriques pénétrant dans la peau en fonction de la température de solubilité des fluorocarbones ou des mélanges de fluorocarbones utilisés.

8. Préparation suivant la revendication 7, caractérisée en ce qu'on sélectionne les phospholipides dans le groupe comprenant des phospholipides naturels comme la lécithine de soja ou la lécithine d'oeuf, ainsi que des phospholipides synthétiques et/ou des phospholipides partiellement hydratés.

9. Préparation suivant la revendication 7 ou 8, caractérisée en ce qu'à côté de la phosphatidylcholine, des lysolécithines sont présentes dans l'intervalle de concentration de 1 à 10% en poids.

10. Préparation suivant l'une des revendications 1 à 9, caractérisée en ce qu'elle comporte en outre des composés pharmaceutiquement actifs choisis parmi les cytostatiques, les cancérostatiques, les immunomodulateurs, les vaccins, les virustatiques, les substances médicamenteuses virucides, les antimycotiques, les héparines, les antibiotiques, les corticoïdes, les agents antiinfectieux, les principes actifs contre l'acné les anesthésiques topiques, les antiphlogistiques, les antihistaminiques, les antipsoriasiques, les analgésiques non stéroïdes, les agents antirhumatismaux, les agonistes et antagonistes de récepteurs d'opiacés, les antagonistes histaminiques, les insulines, les peptides régulateurs et leurs substances inhibitrices, les sédatifs, les hypnotiques, l'acide de romarin ou d'autres principes actifs virucides ou virustatiques qui existent dans les plantes, les vitamines (E, A, B, C), les oligopeptides ou polypeptides, ou qu'elle contient le pigment qu'est la mélanine.

11. Procédé de production d'une préparation favorisant l'irrigation sanguine, caractérisé en ce que des particules mono-orientées fortement magnétiques présentant une intensité de champ coercitif élevée et des granularités de l'ordre de 600 à 1200 nm sont incorporées, par dispersion, dans des substances vectrices et/ou des additifs compatibles sur le plan pharmaceutique ou cosmétique, le cas échéant avec une adjonction d'adjuvants dispersifs usuels pour l'application.

12. Procédé suivant la revendication 11, caractérisé en ce qu'on prépare une formulation cosmétique ou dermatologique avec des proportions des particules mono-orientées fortement magnétiques comprises entre 0,01 et 70% en poids, par rapport à la masse totale de la dispersion.

13. Procédé suivant la revendication 12, pour la production d'une préparation cosmétique ou dermatologique, caractérisé en ce que les particules mono-orientées fortement magnétiques sont incorporées dans la substance vectrice, en combinaison avec des agrégats lamellaires asymétriques se composant de phospholipides ayant une teneur en phosphatidylcholine de l'ordre de 30 à 99% en poids, et avec des fluorocarbones chargés en oxygène dans la plage de 0,2 à 100% (poids/volume).

14. Utilisation d'un système de fabrication d'une préparation favorisant l'irrigation sanguine, le système comprenant une substance vectrice compatible pharmaceutiquement, et des particules mono-orientées fortement magnétiques s'y trouvant dispersées et présentant une intensité de champ coercitif élevée et des granularités de l'ordre de 600 à 1200 nm.

15. Utilisation d'un système de fabrication d'une préparation cosmétique ou dermatologique favorisant l'irrigation sanguine, à appliquer sur la peau, ladite préparation contenant des particules mono-orientées fortement magnétiques présentant une intensité de champ coercitif élevée et des granularités de l'ordre de 600 à 1200 nm, dispersées dans des substances vectrices et éventuellement des additifs compatibles, sur le plan cosmétique ou dermatologique, le système étant distribué dans un support de type usuel pour l'application cosmétique ou dermatologique, tel qu'onguents, crèmes, lotions, eau, extraits alcooliques, pâtes, gels, poudres, teintures ou se présentant, le cas échéant, sur un pansement, un emplâtre ou sous forme de spray.

16. Utilisation suivant la revendication 14 ou 15, la préparation contenant, outre les particules mono-orientées fortement magnétiques présentant une intensité de champ coercitif élevée et des granularités de l'ordre de 600 à 1200 nm, pour contrôler également l'alimentation de la peau en oxygène, et en guise de porteurs d'oxygène lamellaires asymétriques, des phospholipides ayant une teneur en phosphatidylcholine de 30 à 99% en poids, et des fluorocarbones dans l'intervalle de 0,2 à 100% poids/volume, et la pénétration dans la peau étant contrôlée par la structure porteuse des agrégats de phospholipides et la température de solubilité critique des fluorocarbones (dans du n-hexane).

17. Utilisation suivant la revendication 14 à 16, caractérisée en ce qu'un système selon la revendication 1 ou 7 contient, en outre, des substances à activité pharmaceutique.
